# EUROPEAN PATENT APPLICATION

(11) **EP 3 078 326 A1**
(43) Date of publication of application: **12.10.2016**
(21) Application number: 14868106.7
(22) Date of filing: 12.09.2014
(51) Int. Cl.: A61B 5/11

(54) **INFORMATION-PROCESSING APPARATUS, INFORMATION-PROCESSING METHOD, AND PROGRAM**

(30) Priority: 03.12.2013 JP 2013250187
(71) Applicant: Sony Corporation, Tokyo 108-0075 (JP)
(72) Inventor: KATSU, Masanori, Tokyo 108-0075 (JP); KURATA, Masatomo, Tokyo 108-0075 (JP); MATSUZAWA, Sota, Tokyo 108-0075 (JP)
(74) Representative: MFG Patentanwälte Meyer-Wildhagen Meggle-Freund Gerhard PartG mbB
(86) International application number: PCT/JP2014/074257
(87) International publication number: WO 2015/083411

(57) **Abstract**

There is provided an information processing apparatus including a stable section detection unit configured to detect a stable section of a posture of a sensor device that a user wears, based on first sensor data provided from the sensor device, a specific action section detection unit configured to detect a specific action section in which a specific action of the user occurs, based on second sensor data, and a sleep section detection unit configured to detect a sleep section in which the user is in a sleep state, based on a relationship between the stable section and the specific action section.

## Description

### Technical Field

The present disclosure relates to an information processing apparatus, an information processing method, and a program.

### Background Art

Many technologies for analyzing a sleep state, which is a fundamental behavior of a human, have already been proposed. For example, Patent Literature 1 describes a technology for presenting a user with an evaluation of quality of sleep, based on a measurement result of a biological signal during sleep.

### Citation List

### Patent Literature

Patent Literature 1: JP 2013-52165A

### Summary of Invention

### Technical Problem

In recent years, the miniaturization of a sensor device makes it possible for a user to acquire a log of activities while wearing the sensor device routinely as well as during sleep. Incidentally, the technology described in Patent Literature 1 is based on the assumption that the user is in a sleep state. That is, these technologies analyze what the user's sleep state is like, but do not determine whether or not the user is in the sleep state. Hence, it cannot be said that these technologies are sufficient to detect or analyze a user's sleep state along with a user's daily activities.

Therefore, in the present disclosure, there are provided novel and improved information processing apparatuses, information processing methods, and programs that can detect and analyze a user's sleep state along with a user's daily activities.

### Solution to Problem

According to the present disclosure, there is provided an information processing apparatus including a stable section detection unit configured to detect a stable section of a posture of a sensor device that a user wears, based on first sensor data provided from the sensor device, a specific action section detection unit configured to detect a specific action section in which a specific action of the user occurs, based on second sensor data, and a sleep section detection unit configured to detect a sleep section in which the user is in a sleep state, based on a relationship between the stable section and the specific action section.

Additionally, according to the present disclosure, there is provided an information processing method including detecting a stable section of a posture of a sensor device that a user wears, based on first sensor data provided from the sensor device, detecting a specific action section in which a specific action of the user occurs, based on second sensor data, and detecting, by a processor, a sleep section in which the user is in a sleep state, based on a relationship between the stable section and the specific action section.

In addition, according to the present disclosure, there is provided a program for causing a computer to execute a function of detecting a stable section of a posture of a sensor device that a user wears, based on first sensor data provided from the sensor device, a function of detecting a specific action section in which a specific action of the user occurs, based on second sensor data, and a function of detecting a sleep section in which the user is in a sleep state, based on a relationship between the stable section and the specific action section.

### Advantageous Effects of Invention

According to the present disclosure described above, it is possible to detect or analyze a user's sleep state along with a user's daily activities.

Note that the effects described above are not necessarily limited, and along with or instead of the effects, any effect that is desired to be introduced in the present specification or other effects that can be expected from the present specification may be exhibited.

### Brief Description of Drawings

[FIG. 1] FIG. 1 is a block diagram showing a schematic configuration of a system according to a first embodiment of the present disclosure.
[FIG. 2] FIG. 2 is a diagram for describing a principle of sleep section detection in the first embodiment of the present disclosure.
[FIG. 3] FIG. 3 is a block diagram showing a schematic functional configuration of an information processing apparatus, which can perform sleep section detection, according to a first embodiment of the present disclosure.
[FIG. 4] FIG. 4 is a diagram for describing a processing flow of sleep section detection in the first embodiment of the present disclosure.
[FIG. 5] FIG. 5 is a block diagram showing a schematic configuration of a system according to a second embodiment of the present disclosure.
[FIG. 6] FIG. 6 is a diagram for describing a selective use of detectors in a fourth embodiment of the present disclosure.
[FIG. 7] FIG. 7 is a block diagram showing a hardware configuration of an information processing apparatus according to an embodiment of the present disclosure.

### Description of Embodiments

Hereinafter, preferred embodiments of the present disclosure will be described in detail with reference to the appended drawings. Note that, in this specification and the drawings, elements that have substantially the same function and structure are denoted with the same reference signs, and repeated explanation is omitted.

Note that description will be provided in the following order.
1. First Embodiment
   1-1. Configuration of System
   1-2. Principle of Sleep Section Detection
   1-3. Functional Configuration of Information Processing Apparatus
   1-4. Processing Flow of Sleep Section Detection
2. Second Embodiment
3. Third Embodiment
4. Fourth Embodiment
5. Example Application of Detection Result
6. Hardware Configuration
7. Supplemental Remarks

### (1. First Embodiment)

### (1-1. Configuration of System)

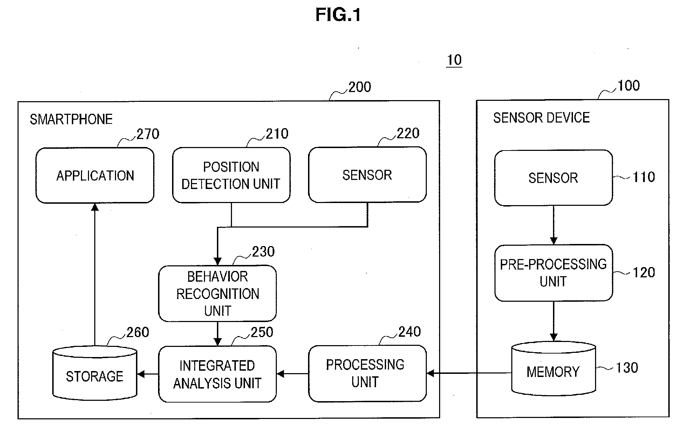

FIG. 1 is a block diagram showing a schematic configuration of a system according to a first embodiment of the present disclosure. Referring to FIG. 1, a system 10 includes a sensor device 100 and a smartphone 200. The sensor device 100 includes a sensor 110, a pre-processing unit 120, and a memory 130. The smartphone 200 includes a position detection unit 210, a sensor 220, a behavior recognition unit 230, a processing unit 240, an integrated analysis unit 250, a storage 260, and an application 270.

### (Sensor Device)

The sensor device 100 is a wearable device. The sensor device 100 is directly attached to a user, for example, by being wound around a user's wrist, angle, or finger. Alternatively, the sensor device 100 may be indirectly attached to the user by being fixed to a garment by using a clip or the like. Note that although the sensor device 100 is a device that is attached to the user, the sensor device 100 need not be always necessarily attached to the user. For example, the sensor device 100 may be detached while the user is taking a bath or is getting ready. Therefore, as described below, a case where a detection value of an acceleration sensor included in the sensor 110 is not changed may include a case where the user wears the sensor device 100 and is in a stable posture and a case where the sensor device 100 is detached and left somewhere.

As described above, the sensor device 100 includes, in addition to the sensor 110, a processor (realizing the pre-processing unit 120) that processes sensor data acquired by the sensor 110, a storage device (realizing the memory 130) that stores the sensor data or the processed data, and a communication device (not shown) that transmits the sensor data or the processed data to the smartphone 200. The sensor device 100 including the processor may be an information processing apparatus according to an embodiment of the present disclosure. The information processing apparatus can be realized using a hardware configuration to be described below. In the following, the respective elements of the sensor device 100 will be described.

The sensor 110 includes various types of sensors provided in the sensor device 100 and senses a behavior of the user wearing the sensor device 100. For example, the sensor 110 includes an acceleration sensor. A posture or a body movement of the user wearing the sensor device 100 can be specified based on an acceleration sensor detection value acquired by the acceleration sensor. In addition, the sensor 110 may include other sensors, such as an angular velocity sensor, a gyro sensor, an optical sensor, a sound sensor, or an atmospheric pressure sensor. Detection values of these sensors also can be used for specifying the posture or the body movement of the user. Furthermore, the sensor 110 may include a camera for acquiring an image of the user, or a microphone for acquiring a voice uttered by the user. The sensor data acquired by the sensor 110 may be temporarily stored in the memory 130 after processing by the pre-processing unit 120, or may be directly stored in the memory 130 without undergoing the pre-processing.

The pre-processing unit 120 performs the pre-processing on the sensor data acquired by the sensor 110. For example, the pre-processing unit 120 extracts a feature amount from the sensor data. Also, the pre-processing unit 120 may remove noise contained in the sensor data and resample the sensor data. Note that, in the present embodiment, it is desirable that the processing of the sensor data by the pre-processing unit 120 can restore raw data of the sensor data or data close to the raw data from the processed data. In addition, in some examples of the present embodiment, all or part of analysis processing performed in the processing unit 240 of the smartphone 200 may be performed by the pre-processing unit 120.

The memory 130 stores the sensor data that has undergone the processing by the pre-processing unit 120, or the raw data of the sensor data that has not undergone the pre-processing. The data stored in the memory 130 is transmitted to the smartphone 200 by the communication device (not shown). The communication between the sensor device 100 and the smartphone 200 can use, for example, radio communication such as Bluetooth (registered trademark).

### (Smartphone)

The smartphone 200 is a terminal device that the user carries separately from the sensor device 100. The smartphone 200 is realized by, for example, a hardware configuration of an information processing apparatus to be described below. Also, the smartphone 200 may be replaced with other terminal device that can implement a similar function, such as a tablet terminal, for example. In the following, the respective elements of the smartphone 200 will be described.

The position detection unit 210 is realized by, for example, a global positioning system (GPS) receiver. In this case, the position detection unit 210 acquires position information of the smartphone 200 by receiving a radio wave from a satellite. Alternatively, the position detection unit 210 may be realized by a communication device that performs radio communication such as Wi-Fi. In this case, the position detection unit 210 acquires the position information of the smartphone 200 based on position information of a radio-communication base station or a received state of radio wave from the base station.

Similar to the sensor 110 of the sensor device 100, the sensor 220 may include various sensors, such as an acceleration sensor, an angular velocity sensor, a gyro sensor, an optical sensor, a sound sensor, an atmospheric pressure sensor, a camera, and a microphone. In the present embodiment, unlike the sensor device 100, the smartphone 200 may be placed on a desk while the user is working, the smartphone 200 may be put in a bag while the user is moving, or the smartphone 200 may be placed near a bed while the user is asleep. Therefore, the sensor 110 of the sensor device 100 is mainly used to acquire information indicating the posture or the body movement of the user. On the other hand, unlike the sensor device 100 requiring a reduction in size and weight in order for attachment to the user, some degree of volume or weight is allowable to the smartphone 200. Hence, the sensor 220 can include more types of sensors than the sensor 110 or can include sensors having higher accuracy than the sensor 110.

The behavior recognition unit 230 is realized by, for example, a processor. The behavior recognition unit 230 recognizes user behavior based on the position information of the smartphone 200 detected by the position detection unit 210 and/or the sensor data provided from the sensor 220. For the recognition of the behavior using the position information and the sensor data, various known technologies can be used, and thus, a detailed description thereof will be omitted herein. As described below, in the present embodiment, the user behavior recognized by the behavior recognition unit 230 is used in combination with data analysis result from the sensor device 100. In another example, the user behavior recognized by the behavior recognition unit 230 may be used by the application 270 or the like, separately from the data analysis result from the sensor device 100.

The processing unit 240 is realized by, for example, a processor. The processing unit 240 performs analysis processing on the data received from the sensor device 100 by the communication device (not shown). For example, the processing unit 240 detects a sleep section, in which the user wearing the sensor device 100 has been in a sleep state, by analyzing the sensor data of the sensor 110. Incidentally, details of the processing of sleep section detection will be described below. As described above, all or part of the analysis processing performed by the processing unit 240 may be realized in a distributed manner by the pre-processing unit 120 of the sensor device 100.

The integrated analysis unit 250 is realized by, for example, a processor. The integrated analysis unit 250 integrates the user behavior recognition result by the behavior recognition unit 230 and the sleep section detection result by the processing unit 240. In this way, by integrating the user behavior recognition result based on different data, for example, the user behavior that is missing in the respective results can be complemented, the accuracy of the result can be improved by adjusting the results to match with each other, and false detection can be prevented. The user behavior recognition result integrated by the integrated analysis unit 250 is stored in the storage 260. Aside from this, the user behavior analysis result by the behavior recognition unit 230 or the sleep section detection result by the processing unit 240 also may be stored in the storage 260.

The storage 260 is realized by, for example, a memory or a storage device. The user behavior recognition result acquired by the processing of the sensor device 100 and the smartphone 200 is accumulated in the storage 260. For example, a user behavior log, a position information log, number of steps, a sleep time log, and the like are accumulated in the storage 260. Such data is generated based on, for example, the behavior recognition result by the behavior recognition unit 230 and the sleep section detection result by the processing unit 240. The logs, which are temporarily or persistently accumulated in the storage 260, are used by the application 270.

The application 270 is application software that is executed by the smartphone 200 and uses the user behavior log. The application 270 is executed by the processor of the smartphone 200 and uses an output device such as a display or a speaker, or an input device such as a touch panel as necessary. In addition, the application 270 may transmit a control signal to an external device through the communication device or transmit information toward other users. A specific example of the application 270 will be described below.

### (1-2. Principle of Sleep Section Detection)

Next, the principle of the sleep section detection according to the first embodiment will be described.

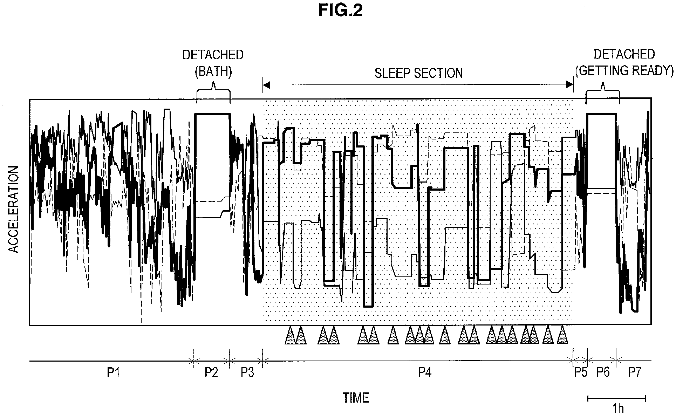

FIG. 2 is a diagram for describing the principle of the sleep section detection in the first embodiment of the present disclosure. FIG. 2 shows a time change in a detection value of a 3-axis acceleration sensor included in the sensor 110 of the sensor device 100. In the following, the time divided into sections P1 to P7 in the shown example will be described.

In the sections P1, P3, P5, and P7, the detection value of the acceleration sensor is continuously and greatly changed. In this section, it is estimated that the user conducts various activities in a waking state. On the other hand, in the sections P2 and P6, the detection value of the acceleration sensor is constant without change. Also, in the section P4, the detection value of the acceleration sensor is constant for a while, is then changed in a short time, and is constant again for a while. In the section P4, such a change is repeated.

As described below, the case where the detection value of the acceleration sensor is not changed may include the case where the user wears the sensor device 100 and is in the stable posture and the case where the sensor device 100 is detached and left somewhere. In order for detecting the sleep section of the user, it is necessary to distinguish these cases from each other. Here, the present inventors focused on the fact that, in a case where the user is in the sleep state, the body of the user is not stationary for quite a while, but the posture of the user is occasionally changed and is then stationary again. Such a user behavior is observed as, for example, turning-over. The turning-over is a very natural behavior found in many users.

In the present embodiment, the sleep state of the user is defined as a state in which first sections in which the posture of the user remains unchanged (section in which the detection value of the acceleration sensor is constant without change) and second sections in which the posture of the user is converted (section in which the detection value of the acceleration sensor is changed and which is sufficiently shorter than the first period) are repeated. On the contrary, in a case where the user detaches the sensor device 100, it is considered that after the detection value of the acceleration sensor is constant without change for quite a while, the sensor device 100 is attached again to the user and thus the continuous change of the detection value is resumed. Therefore, the case where the user wears the sensor device 100 and is in the stable posture and the case where the sensor device 100 is detached and left somewhere can be identified by the presence or absence of the second section before and after the first section.

Referring again to FIG. 2, based on such knowledge, in the sections P2 and P6, the detection value of the acceleration sensor is constant without change, but in the sections (sections P1, P3, P5, and P7) in which the detection value is changed before and after the sections P2 and P6, all detection values are continuously changed, and also, the length of these sections is not always shorter than the sections P2 and P6. Therefore, these sections are estimated as not the case where the user wears the sensor device 100 and is in the stable posture but the case where the sensor device 100 is detached and left somewhere.

On the other hand, in the section P4, first sections in which the detection value of the acceleration sensor is constant without change and second sections in which the detection value of the acceleration sensor is changed between the first sections in a relatively short time are repeated. Therefore, in the section P4, it is estimated that the user wears the sensor device 100 and is in the stable posture.

Note that, strictly speaking, the case where the user wears the sensor device 100 and is in the stable posture is not the same as the case where the user in the sleep state. However, in the present embodiment, the case where the user wears the sensor device 100 and is in the stable posture is considered as the case where the user is in the sleep state. For example, even in a case where the user is still watching a TV or reading a book, the posture of the user is stable, but it is considered that a stable time is shorter than the case where the user is in a sleep state, or the posture of the user is slightly changed. In these cases, for example, in the sensor data analysis processing, an average value or a variance value of acceleration for determining that the posture of the sensor device is in a stable state is appropriately set, and the first section can be recognized and identified only when the stable state is continued over a predetermined length. Alternatively, in a case where a part of the body of the user wearing the sensor device 100 is known, whether the user is in a sleep state or in a stable state other than the sleep state can be identified according to the direction of the sensor device 100. Also, it may be considered that the user is lying down but is in a non-sleep state. However, in this case, it may be considered that the user is in a sleep state.

So far, the principle of the sleep section detection according to the present embodiment has been described. In the following, a functional configuration of an information processing apparatus for detecting a sleep section of a user based on the above-described principle will be further described.

### (1-3. Functional Configuration of Information Processing Apparatus)

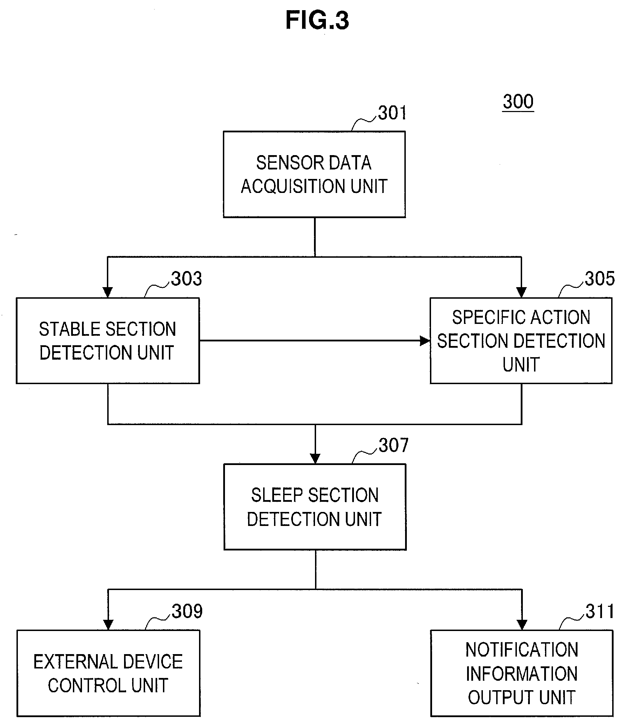

FIG. 3 is a block diagram showing a schematic functional configuration of an information processing apparatus, which can perform sleep section detection, according to a first embodiment of the present disclosure. Referring to FIG. 3, an information processing apparatus 300 includes a sensor data acquisition unit 301, a stable section detection unit 303, a specific action section detection unit 305, a sleep section detection unit 307, an external device control unit 309, and a notification information output unit 311. In the present embodiment, the information processing apparatus 300 is realized by a processor included in a sensor device 100 or a smartphone 200.

For example, the information processing apparatus 300 may be realized in the smartphone 200. In this case, when corresponding to the elements of the smartphone 200 shown in FIG. 1, the sensor data acquisition unit 301, the stable section detection unit 303, the specific action section detection unit 305, and the sleep section detection unit 307 correspond to the processing unit 240, and the external device control unit 309 and the notification information output unit 311 correspond to the application 270. Also, the sleep section detection unit 307 may correspond to both the processing unit 240 and the integrated analysis unit 250.

In addition, for example, the information processing apparatus 300 may be realized in a distributed manner by the sensor device 100 and the smartphone 200. In this case, when corresponding to the elements of the sensor device 100 and the smartphone 200 shown in FIG. 1, the sensor data acquisition unit 301, the stable section detection unit 303, the specific action section detection unit 305, and the sleep section detection unit 307 correspond to the processing unit 120 of the sensor device 100, and the external device control unit 309 and the notification information output unit 311 correspond to the application 270 of the smartphone 200.

Note that the elements for realizing the information processing apparatus 300 are not limited to the above-described example, and a variety of other examples are possible within the scope obvious from the description of the present specification to those skilled in the art. For example, the entire functions of the information processing apparatus 300 may be realized in the sensor device 100. Also, for example, in a case where the information processing apparatus 300 is realized in a distributed manner by the sensor device 100 and the smartphone 200, the sleep section detection unit 307 is realized by the pre-processing unit 120 of the sensor device 100 and the processing unit 240 and/or the integrated analysis unit 250 of the smartphone 200.

In the following, the respective elements of the information processing apparatus 300 will be further described.

The sensor data acquisition unit 301 is realized by a processor as a software interface that acquires sensor data provided from the sensor device 100. The sensor data may be data that is output by various sensors, such as an acceleration sensor, an angular velocity sensor, a gyro sensor, an optical sensor, a sound sensor, an atmospheric pressure sensor, a camera, or a microphone, which is included in the sensor 110, and is processed by the pre-processing unit 120 as necessary. In addition, the sensor data, which is acquired by the sensor data acquisition unit 301, includes first sensor data to be used by the stable section detection unit 303, and second sensor data to be used by the specific action section detection unit 305. Such data may be the same data, for example, the detection value of the common acceleration sensor, or may be different data.

The stable section detection unit 303 detects a stable section of the posture of the sensor device 100, based on the first sensor data acquired by the sensor data acquisition unit 301. The stable section is a section that becomes a candidate for the first section described above with reference to FIG. 2. As in the present embodiment, for example, the stable section of the posture of the sensor device 100 is defined as the section in which the posture of the sensor device 100 has been unchanged. For example, in a case where the first sensor data includes the detection value of the acceleration sensor, the stable section detection unit 303 detects the stable section as the section in which the detection value is unchanged. The stable section may be limited to a section that is longer than a predetermined time (for example, several minutes). However, as already described above, since the detected stable section is only the stable section of the posture of the sensor device 100, whether or not the user wears the sensor device 100 and is in a stable posture and whether or not the sensor device 100 is detached and left somewhere are not specified by the stable section detection result alone. Therefore, in the present embodiment, specific action section detection is performed by the specific action section detection unit 305 to be described below.

The specific action section detection unit 305 detects a specific action section in which a specific action of a user occurs, based on the second sensor data acquired by the sensor data acquisition unit 301. The specific action section is a section corresponding to the second section described above with reference to FIG. 2. In the present embodiment, similar to the first sensor data, the second sensor data includes the detection value of the acceleration sensor. Also, in the present embodiment, the specific action section is a section in which the posture of the user is changed. Also, in the stable section, it is necessary to consider the probability that the sensor device 100 is detached, but in the sections (including the specific action section) other than the stable section, the probability that the sensor device 100 is detached is negligibly low. Therefore, in the present embodiment, in the sections other than the stable section, the change in the posture of the sensor device 100, which is indicated by the second sensor data, can be considered as the change in the posture of the user.

More specifically, for example, the specific action section detection unit 305 may detect the specific action section on the condition that (1) the specific action occurs subsequently to the stable section having a certain length (for example, several minutes or more), (2) the detection value of the acceleration sensor is changed in a sufficiently shorter time than the stable section, and (3) another stable section in which the detection value of the acceleration sensor is stable at a different value from the previous stable section occurs subsequently.

The sleep section detection unit 307 detects the sleep section in which the user is in a sleep state, based on a relationship between the stable section detected by the stable section detection unit 303 and the specific action section detected by the specific action section detection unit 305. In the present embodiment, in a case where the stable section and the specific action section are repeated, the sleep section detection unit 307 detects a sleep section including the stable section and the specific action section. In the example described above with reference to FIG. 2, the section P4 is the sleep section, but in the section P4, the first sections (stable sections) in which the detection value of the acceleration sensor is constant without change and the second sections (specific action sections) in which the detection value of the acceleration sensor is changed between the first sections in a relatively short time are repeated.

More specifically, for example, the sleep section detection unit 307 may detect, as the sleep section, the sections from (1) the first stable section occurring subsequently to the non specific action section through (2) the specific action section subsequent to the first stable section and (3) a middle stable section interposed between the previous and next specific action sections to (4) the last stable section which occurs subsequently to the last specific action section and to which the non specific action section is subsequent. In this case, a start time of the stable section of (1) can be specified as a sleep start time of the user, and an end time of the stable section of (4) can be specified as a sleep end time of the user.

Also, by using the function of the integrated analysis unit 250 of the smartphone 200, the sleep section detection unit 307 may determines the sleep section in consideration of matching with the user behavior recognized based on the position information or the sensor data by the behavior recognition unit 230. In this case, the sleep section detection unit may detect the sleep section further based on the user behavior that is recognized based on the sensor data different from the first and second sensor data.

Both the external device control unit 309 and the notification information output unit 311, which are to be described below, have a functional configuration to use the sleep section detection result by the sleep section detection unit 307. In such a functional configuration, either or both of the external device control unit 309 and the notification information output unit 311 may be included in the information processing apparatus 300. Also, in the present embodiment, since the method of using the sleep section detection result is not particularly limited, a functional configuration different from both the external device control unit 309 and the notification information output unit 311 may be present for using the sleep section detection result. Also, the sleep section detection result by the sleep section detection unit 307 may be stored in the storage as it is and may be used by a separate device from the information processing apparatus 300.

In a case where the sleep section of the user is detected by the sleep section detection unit 307, the external device control unit 309 outputs a control signal of the external device. For example, in a case where the start of the sleep section is detected, the external device control unit 309 outputs a control signal for stopping or pausing the external device. Also, as is apparent from the above description, the sleep section detection by the sleep section detection unit 307 is enabled for the first time through the specific action section detection occurring afterwards. Therefore, in the present embodiment, the external device control unit 309 cannot transmit the control signal in real time when the sleep state of the user is started. Therefore, in the present embodiment, the control signal transmitted by the external device control unit 309 may be transmitted so as to stop or pause the external device, for example, a TV or an air conditioner (according to temperature), which is considered as unnecessary when the user enters a sleep state.

Alternatively, the external device control unit 309 may output a control signal for causing the external device to set an alarm based on a start time of the sleep section. The external device used herein is merely an external device with respect to the device indicated as the information processing apparatus 300 in FIG. 3. Therefore, for example, in a case where the information processing apparatus 300 is realized in the smartphone 200, other functional elements of the smartphone 200 (including those realized by the processor as in the information processing apparatus 300) also may correspond to the external device (the same applies to the case where the information processing apparatus 300 is realized by the sensor device 100). Therefore, the external device control unit 309 may be realized by, for example, the processor of the smartphone 200, and may transmit the control signal with respect to the alarm function realized by the processor of the same smartphone 200. By automatically setting the alarm based on the start time of the sleep time, for example, it is possible to prevent the user from oversleeping.

The notification information output unit 311 outputs information for notifying other user of the sleep section detection result by the sleep section detection unit 307. For example, the notification information output unit 311 outputs information indicating a start, a continuation, or an end of the sleep section. In this case, for example, it is possible to notify friends on social media that the user wakes up, has slept, or is sleeping. This notification may be a push type notification or may be a pull type notification. In the case of the pull type notification, for example, a sleep state can be represented by an icon of a user expressed in a virtual space, and other user can view the virtual space and recognize that the user is sleeping or awake.

Alternatively, the notification information output unit 311 may output information indicating the length of the sleep section in a predetermined period. In this case, for example, the lack of sleep may be notified to friends according to whether the use's sleeping time per day is more than or less than an average. As in the above-described example, the notification may be output through the virtual space, and rings may be superimposed on the actual user's face by using augmented reality (AR) technology.

Another example of the processing of the external device control unit 309 and the notification information output unit 311 will be described as an example application of the sleep section detection result.

### (1-4. Processing Flow of Sleep Section Detection)

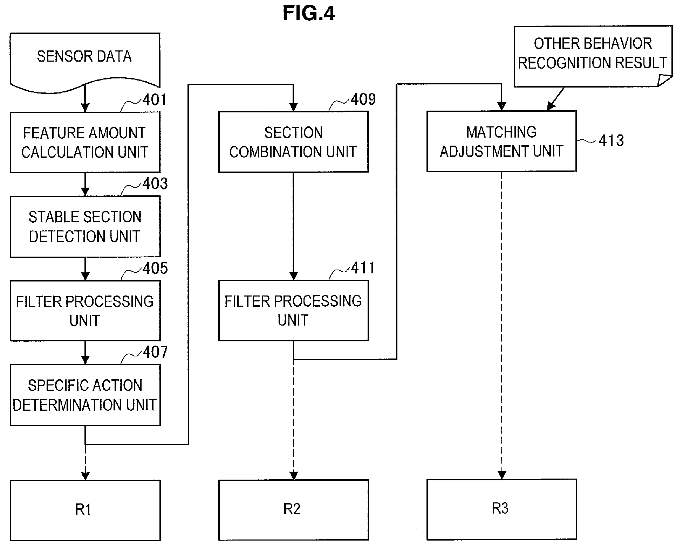

FIG. 4 is a diagram for describing a processing flow of the sleep section detection in the first embodiment of the present disclosure. The processing flow shown in FIG. 4 corresponds to the functional configuration of the information processing apparatus 300 described above with reference to FIG. 3. That is, a series of processing performed by a feature amount calculation unit 401 to a matching adjustment unit 413 may be expressed from a viewpoint different from the functions of the respective elements of the information processing apparatus 300. Therefore, the series of processing is performed by the processor of the sensor device 100 or the smartphone 200. In the following, the processing of the respective elements of the processing flow will be further described.

The feature amount calculation unit 401 calculates feature amount from sensor data. Here, the feature amount to be calculated may be one that can be restored to raw data or data close to the raw data. For example, in a case where the sensor data is data of the acceleration sensor, the feature amount calculation unit 401 may calculate an average value or a variance value of acceleration based on a predetermined time (for example, one minute) as time unit.

The stable section detection unit 403 detects the stable section in which the posture of the user is estimated to be in the stable state, based on the feature amount calculated from the sensor data by the feature amount calculation unit 401. As described above, since the detected stable section is only the stable section of the posture of the sensor device 100, whether or not the user wears the sensor device 100 and is in a stable posture and whether or not the sensor device 100 is detached and left somewhere are not specified by the stable section detection result alone.

A filter processing unit 405 performs filter processing on the stable section detected by the stable section detection unit 403. For example, the filter processing unit 405 filters the detected stable section based on the length of the stable section. More specifically, in a case where the length of the section is equal to or less than a predetermined time (for example, five minutes), the filter processing unit 405 excludes the corresponding section from the stable section. When considering that the stable section is used for detecting the sleep section of the user, a sleep section having a short time may also exist. However, in such a case, for the reason that the stable section does not include the above-described specific action section, it is difficult to distinguish the stable section from a pseudo-stable section occurring for a reason other than sleep. Therefore, in the present embodiment, the section having a short time is excluded from the stable section.

The specific action determination unit 407 detects the specific action section in which the specific action of the user occurs, based on the feature amount calculated from the sensor data by the feature amount calculation unit 401. Furthermore, the specific action determination unit 407 determines whether or not the stable section is the sleep section, based on a relationship between the specific action section and the stable section detected by the stable section detection unit 403 and filtered by the filter processing unit 405. More specifically, in a case where a specific action section is present subsequently to before or/and after the stable section, the specific action determination unit 407 may specify the corresponding stable section and the specific action section subsequent to the stable section as the sleep section.

So far, a result R1 indicating the sleep section is acquired by the processing of the feature amount calculation unit 401, the stable section detection unit 403, the filter processing unit 405, and the specific action determination unit 407. In the result R1, it can be said that the sleep section is specified based on the relationship between the pure stable section and the specific action section, without applying the filter with respect to the sleep section detection result, except that the excessively short stable section is excluded by the filter processing unit 405. The result R1 may be used as an input of processing of a section combination unit 409 to be described below or may be output as it is.

The section combination unit 409 combines sections having a close gap in the sleep section specified by the result R1. More specifically, for example, in a case where a gap between the sleep sections is equal to or less than a predetermined time (for example, thirty minutes), the section combination unit 409 combines these sections. For example, as exemplified in the description of the specific action section detection unit 305, in a case where the condition of the specific action section is that the detection values of the acceleration sensor are stable at different values before and after the corresponding section, the section in which the posture returns to the same posture as a result of changing the posture by the user's turning-over and a movement similar thereto is detected as the specific action section. However, such a section also may be included in the sleep section of the user, for example, in a case where the section occurs in a sufficiently short time as compared with the stable section.

The filter processing unit 411 performs filter processing on the stable section after combination by the section combination unit 409. For example, the filter processing unit 411 filters the combined stable section based on the length of the stable section. More specifically, in a case where a length of a sleep section candidate is equal to or less than a predetermined time (for example, thirty minutes), the filter processing unit 411 excludes the corresponding section from the stable section. It is likely that the sleep section that does not reach a sufficient length even when combined will be, for example, a falsely detected stable section occurring for a reason other than sleep. Also, according to the method of using the detection result, there is no problem even though a short sleep section such as a snooze or a nap is not detected. Therefore, in the present embodiment, the filter processing unit 411 excludes the section that does not reach a sufficient length.

In a case where the filtering based on the length of the section is performed in the filter processing unit 405 and/or the filter processing unit 411, a predetermined time that becomes a threshold value is arbitrarily set and is not limited to the above-described example (five minutes and thirty minutes). However, in a case where the filtering is performed by the filter processing units 405 and 411 as in the above-described example, the threshold time by the filter processing unit 411 (which is used for excluding the excessively short sleep section even when combined) is longer than the threshold time by the filter processing unit 405 (which is used for excluding the excessively short stable section).

So far, a result R2 indicating the sleep section is acquired by the processing of the section combination unit 409 and the filter processing unit 411 with respect to the result R1. In the result R2, the sleep sections that are specified by the result R1 and have a close gap are combined (section combination unit 409), and furthermore, the sleep sections that do not reach a sufficient length even when combined are excluded (filter processing unit 411). Therefore, in the result R2, it can be said that a weak filter using the gap or length indicated by the detection result itself is applied with respect to the sleep section detection result. The result R2 may be used as an input of a matching adjustment unit 413 to be described below or may be output as it is.

The matching adjustment unit 413 adjusts a matching with a behavior recognition result by another method in the sleep section specified by the result R2. More specifically, for example, the matching adjustment unit 413 adjusts the matching between the result of the behavior recognition performed by the behavior recognition unit 230, based on the detection result of the position detection unit 210 and the sensor 220 of the smartphone 200, and the sleep section specified by the result R2. For example, in the sleep section specified by the result R2, in a case where the behavior recognition unit 230 recognizes a behavior of "walking" (longer-distance walking, not walking in the house), either or both of the sleep section or the behavior recognition result is considered as an error.

In such a case, for example, the matching adjustment unit 413 may fixedly prioritize either the sleep section or the behavior recognition result. For example, in a case where the sleep section is preferred, the behavior such as "walking" recognized in the sleep section is ignored. On the contrary, in a case where the behavior recognition result is preferred, the sleep section in which the behavior such as "walking" is recognized is determined as being falsely detected. Alternatively, the matching adjustment unit 413 may select the result employed based on the reliability degree of each of the sleep section and the behavior recognition result. Also, in a case where the sleep section detection result and the behavior recognition result conflict each other, the matching adjustment unit 413 may set the user's behavior in the corresponding section as "unknown".

A result R3 indicating the sleep section is acquired through the processing of the matching adjustment unit 413 as described above. In the result R3, it can be said that a stronger filter is applied to the sleep section specified by the result R2 in consideration of the matching with the behavior recognition result by another method. The result R3 is output for use in the application 270 of the smartphone 200. As described above, the result R1 and/or the result R2 also are output along with the result R3 or instead of the result R3. Therefore, in the present embodiment, for example, the result R1 may be output and the processing of the section combination unit 409 to the matching adjustment unit 413 may not be performed. The result R2 may be output and the processing of the matching adjustment unit 413 may not be performed.

### (2. Second Embodiment)

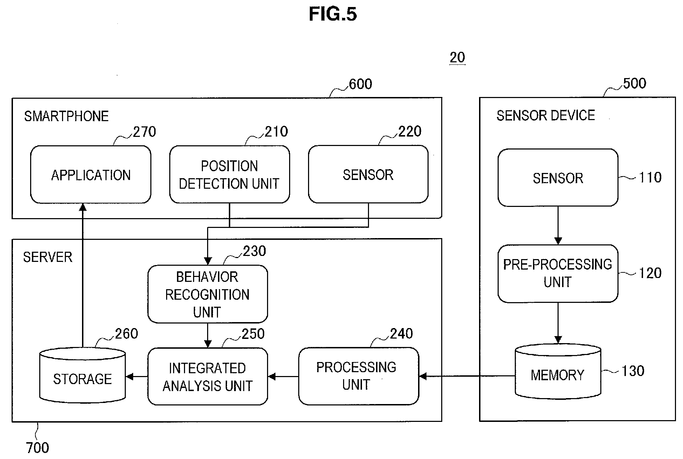

FIG. 5 is a block diagram showing a schematic configuration of a system according to a second embodiment of the present disclosure. Referring to FIG. 5, a system 20 includes a sensor device 500, a smartphone 600, and a server 700. The sensor device 500 includes a sensor 110, a pre-processing unit 120, and a memory 130. The smartphone 200 includes a position detection unit 210, a sensor 220, and an application 270. The server 700 includes a behavior recognition unit 230, a processing unit 240, an integrated analysis unit 250, and a storage 260.

### (Sensor Device)

The sensor device 500 is a wearable device as in the sensor device 100 according to the first embodiment. The sensor 110, the processing unit 120, and the memory 130, which are included in the sensor device 100, are similar to those of the first embodiment. However, the sensor device 500 differs from the sensor device 100 according to the first embodiment, in that data stored in the memory 130 is transmitted to the server 700 directly or indirectly through a communication device (not shown). The sensor device 500 may transmit data to the smartphone 600 by using wireless communication such as radio communication such as Bluetooth (registered trademark), and the communication device (not shown) of the smartphone 600 may transmit data to the server 700 through a network. Alternatively, the sensor device 500 can directly communicate with the server 700 through the network.

### (Smartphone)

As in the smartphone 200 according to the first embodiment, the smartphone 600 is a terminal device that is carried by the user separately from the sensor device 500. The position detection unit 210, the sensor 220, and the application 270, which are included in the smartphone 600, are elements similar to those of the first embodiment. On the other hand, in the present embodiment, the smartphone 600 does not include processing elements such as the behavior recognition unit 230, the processing unit 240, and the integrated analysis unit 250. That is, in the present embodiment, the smartphone 600 includes the processor, but the above-described elements are not realized by the processor. The detection results by the position detection unit 210 and the sensor 220 are transmitted to the server 700 through the network by the communication device (not shown). Also, data, which is used by the application 270, is transmitted from the server 700 through the network by the communication device (not shown). Furthermore, as described above, the smartphone 600 may stop transmitting data from the sensor device 500 to the server 700.

### (Server)

The server 700 is realized by one information processing apparatus or a plurality of information processing apparatuses on the network. The behavior recognition unit 230, the processing unit 240, the integrated analysis unit 250, and the storage 260, which are included in the server 700, are elements similar to those included in the smartphone 200 according to the first embodiment. Since the functions of these elements are substantially the same as those of the first embodiment, a detailed description thereof will be omitted.

As described above, in the present embodiment, some functions realized by the smartphone 200 according to the first embodiment are realized by the server 700. In the present embodiment, the server 700 is an information processing apparatus that performs the sleep section detection. The configuration of the system including the server according to the embodiment of the present disclosure is not limited to the example shown in FIG. 5. For example, some of the behavior recognition unit 230, the processing unit 240, and the integrated analysis unit 250 may be realized by the sensor device 500 or the smartphone 600. In this case, for example, the device realized by the processing unit 240 is the information processing apparatus according to the embodiment of the present disclosure.

In the present embodiment, in a case where the detection of the sleep section of the user based on the sensor data acquired by the sensor device 500 is performed by the server 700, the smartphone 600 may not be necessarily included in the system. In this case, the behavior recognition unit 230 and the integrated analysis unit 250 may not be included in the server 700, and the data of the sleep section detected based on the sensor data by the processing unit 240 may be stored in the storage 260 as it is.

### (3. Third Embodiment)

Next, a third embodiment of the present disclosure will be described. The present embodiment is similar to the first or second embodiment in terms of the device configuration, but is different in the sleep section detection processing performed by the information processing apparatus 300 described above with reference to FIG. 3. Therefore, in the following, the difference will be mainly described and a detailed description of the points (device configuration) common to the above-described embodiments will be omitted. In the following description of the third and fourth embodiments, the reference signs of the first embodiment (the sensor device 100 and the smartphone 200) are recited, but the similar configuration is possible to the second embodiment (the sensor device 500, the smartphone 600, and the server 700).

In the present embodiment, the stable section detection unit 303 detects a section in which the posture of the sensor device 100 is regularly changed. For example, such a stable section is detected as a section in which the acceleration sensor detection value included in the first sensor data is regularly changed. In a case where the user is in a sleep state, some examples may be considered. One example is a case where the user is lying down on a bed and is substantially stationary and asleep. Another example is a case where the user is asleep while sitting on a chair in a house or a vehicle. The latter case is a so-called "nod off" state. For example, in the latter case, a regular body movement such as up-and-down movement of a head occurs. In the present embodiment, such a state is detected as a sleep state.

More specifically, for example, in a case where the stable section detection unit 303 detects a stable section, the specific action section detection unit 305 detects a section in which an image or a voice of a user shows a characteristic associated with a sleep. In this case, the second sensor data acquired by the sensor data acquisition unit 301 includes the image or the voice of the user that is acquired by the camera or the microphone. For example, the specific action section detection unit 305 may detect, as a specific action section, a section in which it is recognized that the user closes his or her eyes by image data contained in the second sensor data. That the user has closed his or her eyes may be detected by, for example, another sensor detecting a movement of eyes or an electro-oculogram. Also, the specific action section detection unit 305 may detect, as a specific action section, a section in which it is recognized that the user emits a specific sound, based on audio data contained in the second sensor data. Here, such a specific sound is, for example, a sound corresponding to a sleep breath or a snoring.

In the present embodiment, in a case where the stable section detected by the stable section detection unit 303 and the specific action section detected by the specific action section detection unit 305 are concurrent, the sleep section detection unit 307 detects the sleep section including the stable section. That is, in the present embodiment, a section in which the posture of the sensor device 100 is regularly changed in the stable section and the image or the voice of the user shows a characteristic associated with a sleep (closing eyes, making a sleep breath, snoring, or the like)

### (4. Fourth Embodiment)

Next, a fourth embodiment of the present disclosure will be described. The present embodiment is configured by, for example, a combination of the first to third embodiments. In the present embodiment, a plurality of detectors is selectively used for detecting a sleep section of a user.

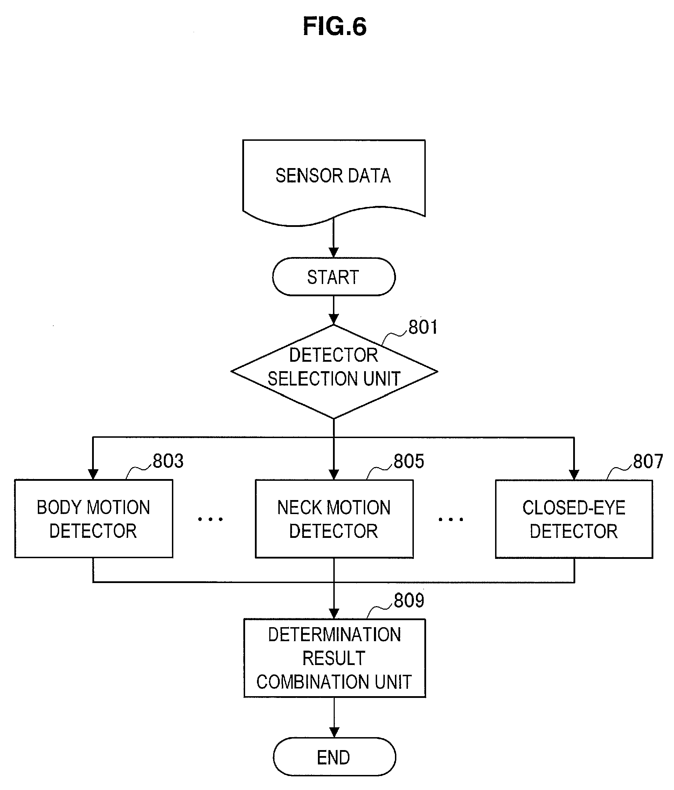

FIG. 6 is a diagram for describing a selective use of detectors in a fourth embodiment of the present disclosure. Referring to FIG. 6, in the present embodiment, when sensor data is input and the sleep section detection processing is started, a detector selection unit 801 selects one or more detectors to be used. In the detectors, for example, there are a body motion detector 703, a neck motion detector 805, and a closed-eye detector 807. The detection results by one or more detector selected among these detectors by the detector selection unit 801 are combined by a determination result combination unit 809 and are output as a sleep section detection result.

The detector selection unit 801 is realized by, for example, the processor of the sensor device 100 or the smartphone 200. The detector selection unit 801 selects one or more detectors to be used, for example, according to whether or not the sensor device 100 is attached to the user, how much a battery residual quantity of the sensor device 100 remains, or what action of the user (including simple position information) is recognized by the behavior recognition unit 230 of the smartphone 200 side. All selectable detectors may be selected.

The body motion detector 803 includes, for example, sensing by the acceleration sensor included in the sensor 110, pre-processing of the detection value in the pre-processing unit 120, and detection of the user's body motion based on the detection value and detection of the sleep section based on that in the processing unit 240 (for example, detection of the sleep section using the detection of the section in which the posture of the user is unchanged). Therefore, in a case where the detector selection unit 801 selects the body motion detector 803, a series of processing is performed for detecting a sleep section based on the detection of the user's body motion as described above.

The neck motion detector 805 includes, for example, sensing by the acceleration sensor included in the sensor 110, pre-processing of the detection value in the pre-processing unit 120, and detection of the user's neck motion based on the detection value and detection of the sleep section based on that in the processing unit 240 (for example, detection of the sleep section using the detection of the section in which the user's neck (head) motion is regular (nod-off section)). Therefore, in a case where the detector selection unit 801 selects the neck motion detector 805, a series of processing is performed for detecting a sleep section based on the detection of the user's neck (head) motion as described above.

The closed-eye detector 807 includes, for example, capturing of an image by the camera included in the sensor 110, pre-processing of image data in the pre-processing unit 120, and detection of whether or not the user closes his or her eyes based on the image data and detection of the sleep section based on that in the processing unit 240 (for example, detection of the sleep section on the condition that the body motion of the user is regular and the user closes his or her eyes). Therefore, in a case where the detector selection unit 801 selects the closed-eye detector 807, a series of processing is performed for detecting a sleep section based on the detection of whether or not the user closes his or her eyes as described above.

As described above, the determination result combination unit 809 combines the detection results by the selected one or more detectors and determines the sleep section. The determination result combination unit 809 is realized as, for example, the processing unit 240 of the smartphone 200. In a case where the selected detector is single, the determination result combination unit 809 may output the detection result of the selected detector as it is. Also, in a case where the selected detector is plural, the determination result combination unit 809 may perform, for example, a logical OR operation on the sleep section detection results by the plurality of selected detectors. In this case, the section detected as the sleep section in any one of the detectors is output as the sleep section. Alternatively, the determination result combination unit 809 may perform a logical AND operation on the sleep section detection results by the plurality of selected detectors. In this case, the sections detected as the sleep section in all the selected detectors are output as the sleep sections.

Alternatively, the determination result combination unit 809 may acquire scores indicating probability of the detection result in the respective selected detectors, weight the detection results, and determine the sleep section based on the sum of the scores. In this case, the respective detectors, for example, the body motion detector 803, the neck motion detector 805, and the closed-eye detector 807, output the scores indicating the probability of the detection result to the determination result combination unit 809.

In the present embodiment, by selectively using the plurality of detectors for detecting the sleep section of the user, for example, the detection using the smartphone 200 may be continued, the sensor to be used may be switched according to the battery residual quantity of the sensor device 100, or the method of detecting an optimal sleep section according to situations may be used, even while the sensor device 100 is detached.

### (5. Example Application of Detection Result)

Next, example application of the sleep section detection result according to the embodiment of the present disclosure will be described. As described above, in the embodiment of the present disclosure, for example, in a case where the start of the sleep section is detected, the sleep section detection result is applied by outputting the control signal of the external device and outputting information for notifying other user of the sleep section detection result. In the following, example application of these sleep section detection result will be described with reference to a more specific example.

### (Control of External Device)

As an example in which the external device control unit 309 of the information processing apparatus 300 output the control signal in a case where the start of the sleep section is detected, there are an example in which a control signal for stopping or pausing the external device as described above is output and an example in which a control signal for causing the external device to set an alarm based on the start time of the sleep section is output.

In addition, for example, in a case where the start of the sleep section is detected, the external device control unit 309 may output a control signal for performing a predetermined operation to the external device. As an example, the external device control unit 309 may output a control signal for performing backup or upload, analysis, or the like with respect to an information processing terminal such as a personal computer. When such processing is performed while the user uses the information processing terminal, there is a probability that hinders the operation of the information processing terminal desired by the user in order for consumption of device resources. Therefore, when assuming that such processing can be performed in a case where the start of the sleep section of the user is detected, the processing can be completed without hindering the operation of the user.

### (Notification of other user)

As an example in which the notification information output unit 311 of the information processing apparatus 300 output information for notifying other user of the sleep section detection result, there are an example in which information indicating a start, a continuation, or an end of the sleep section is output as described above and an example in which information indicating the length of the sleep section in a predetermined period is output.

In addition, for example, in a case where schedule information of the user is acquired and it is determined that the sleep state of the user hinders the execution of the schedule, the notification information output unit 311 may output notification to request a friend to take a behavior that wakes the user (for example, making a phone call). In this case, the external device control unit 309 may output a control signal to the external device (as described above, including other functions realized by the same processor) so as to sound an alarm at the time when the user must wake for the execution of the schedule, and in a case where the user does not wake in spite of the alarm (in a case where the end of the sleep section is not detected), the notification information output unit 311 may output the notification to other user.

Also, for example, the notification information output unit 311 may interwork with other information processing terminal, adjust the schedule according to the wake-up time of the user belonging to a predetermined group such as friends, notify the adjusted schedule to each user, and transmit the notification to request sounding of alarm with respect to an information processing terminal of a user who seems to be late for the adjusted schedule. At this time, a distance from a current position of each user to a place (destination) where a scheduled event is executed may be considered. That is, the output of the notification may be adjusted such that a user near the destination wakes immediately before the start time of the event and a user far from the destination wakes long before the start time.

Also, for example, the notification information output unit 311 may output information to other user on the condition that a certain user has woken (the sleep section has been finished). For example, in a case where teleconference intends to be performed in a certain environment with time difference, when a user participating in the early morning woken, the start of the conference may be notified to other user participating in the day. In this way, the contact or notification is smoothly performed in a case where the user want to do something if the user wakes but does not wake.

As another example application of the sleep section detection result according to the embodiment of the present disclosure, not limited to the external device control unit 309 or the notification information output unit 311, the percentage of users who have woken at the sleeping time in countries or regions may be statistically grasped based on the detection results of the sleep sections of users in the corresponding countries and regions and may be used as indexes of how much the events occurring at that time (for example, midnight sports broadcasting) are attracted. Also, for example, the percentage of sleeping users in a certain region may be statistically grasped based on the detection results of the sleep sections of the users in the corresponding region and be used as the criteria of whether or not infrastructure such as a radio-communication base station is operated in a low power consumption mode.

### (6. Hardware Configuration)

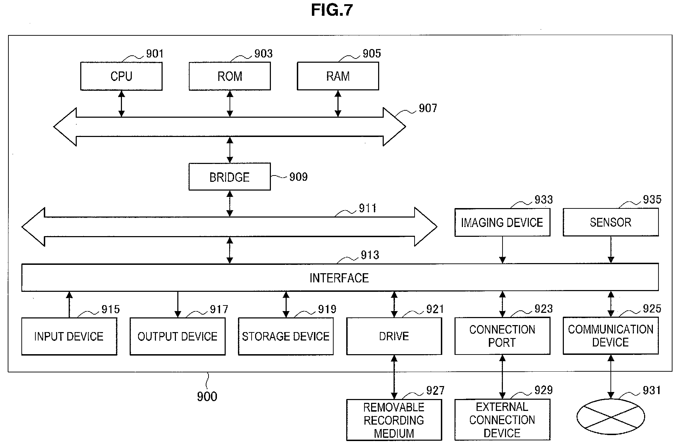

Next, with reference to FIG. 7, a hardware configuration of an information processing apparatus according to an embodiment of the present disclosure will be described. FIG. 7 is a block diagram showing a hardware configuration of the information processing apparatus according to an embodiment of the present disclosure. An information processing apparatus 900 which is shown may achieve the sensor device, the smartphone, and the server in the above described embodiments, for example.

The information processing apparatus 900 includes a central processing unit (CPU) 901, read only memory (ROM) 903, and random access memory (RAM) 905. Further, the information processing apparatus 900 may also include a host bus 907, a bridge 909, an external bus 911, an interface 913, an input device 915, an output device 917, a storage device 919, a drive 921, a connection port 923, and a communication device 925. Furthermore, the information processing apparatus 900 may include an imaging device 933 and a sensor 935 as necessary. The information processing apparatus 900 may also include, instead of or along with the CPU 901, a processing circuit such as a digital signal processor (DSP) or an application specific integrated circuit (ASIC).

The CPU 901 functions as an arithmetic processing unit and a control unit and controls an entire operation or a part of the operation of the information processing apparatus 900 according to various programs recorded in the ROM 903, the RAM 905, the storage device 919, or a removable recording medium 927. The ROM 903 stores programs and arithmetic parameters used by the CPU 901. The RAM 905 primarily stores programs used in execution of the CPU 901 and parameters and the like varying as appropriate during the execution. The CPU 901, the ROM 903, and the RAM 905 are connected to each other via the host bus 907 configured from an internal bus such as a CPU bus or the like. In addition, the host bus 907 is connected to the external bus 911 such as a peripheral component interconnect/interface (PCI) bus via the bridge 909.

The input device 915 is a device operated by a user, such as a mouse, a keyboard, a touch panel, buttons, a switch, and a lever. Also, the input device 915 may be a remote control device using, for example, infrared light or other radio waves, or may be an external connection device 929 such as a cell phone compatible with the operation of the information processing apparatus 900. The input device 915 includes an input control circuit that generates an input signal on the basis of information input by the user and outputs the input signal to the CPU 901. The user inputs various kinds of data to the information processing apparatus 900 and instructs the information processing apparatus 900 to perform a processing operation by operating the input device 915.

The output device 917 is configured from a device capable of visually or aurally notifying the user of acquired information. For example, the output device 917 may be: a display device such as a liquid crystal display (LCD), a plasma display panel (PDP), or an organic electro-luminescence (EL) display; an audio output device such as a speaker or headphones; or a printer. The output device 917 outputs results obtained by the processing performed by the information processing apparatus 900 as video in the form of text or an image or as audio in the form of audio or sound.

The storage device 919 is a device for storing data configured as an example of a storage unit of the information processing apparatus 900. The storage device 919 is configured from, for example, a magnetic storage device such as a hard disk drive (HDD), a semiconductor storage device, an optical storage device, or a magneto-optical storage device. This storage device 919 stores programs to be executed by the CPU 901, various data, and various data obtained from the outside.

The drive 921 is a reader/writer for the removable recording medium 927 such as a magnetic disk, an optical disc, a magneto-optical disk, or a semiconductor memory, and is built in or externally attached to the information processing apparatus 900. The drive 921 reads out information recorded on the attached removable recording medium 927, and outputs the information to the RAM 905. Further, the drive 921 writes the record on the attached removable recording medium 927.

The connection port 923 is a port for allowing devices to directly connect to the information processing apparatus 900. Examples of the connection port 923 include a universal serial bus (USB) port, an IEEE1394 port, and a small computer system interface (SCSI) port. Other examples of the connection port 923 may include an RS-232C port, an optical audio terminal, and a high-definition multimedia interface (HDMI) (registered trademark) port. The connection of the external connection device 929 to the connection port 923 may enable the various data exchange between the information processing apparatus 900 and the external connection device 929.

The communication device 925 is a communication interface configured from, for example, a communication device for establishing a connection to a communication network 931. The communication device 925 is, for example, a wired or wireless local area network (LAN), Bluetooth (registered trademark), a communication card for wireless USB (WUSB), or the like. Alternatively, the communication device 925 may be a router for optical communication, a router for asymmetric digital subscriber line (ADSL), a modem for various communications, or the like. The communication device 925 can transmit and receive signals and the like using a certain protocol such as TCP/IP on the Internet and with other communication devices, for example. The communication network 931 connected to the communication device 925 is configured from a network which is connected via wire or wirelessly and is, for example, the Internet, a home-use LAN, infrared communication, radio wave communication, and satellite communication.

The imaging device 933 is a device which images a real space by use of various members including an image sensor such as a charge coupled device (CCD) or a complementary metal oxide semiconductor (CMOS) and a lens for controlling image formation of a subject on the image sensor, and generates a pickup image. The imaging device 933 may image a still image or a moving image.

The sensor 935 is any of various sensors such as an acceleration sensor, a gyro sensor, a magnetic field sensor, an optical sensor, and a sound sensor. For example, the sensor 935 acquires information related to the state of the information processing apparatus 900 itself, such as the posture of the housing of the information processing apparatus 900, or information related to the peripheral environment of the information processing apparatus 900, such as the brightness or noise around the information processing apparatus 900. Further, the sensor 935 may include a global positioning system (GPS) sensor which measures the latitude, the longitude, and the altitude of the apparatus by receiving a GPS signal.

Heretofore, an example of the hardware configuration of the information processing apparatus 900 has been shown. Each of the structural elements described above may be configured using a general-purpose material, or may be configured from hardware dedicated to the function of each structural element. The configuration may be changed as appropriate according to the technical level at the time of carrying out embodiments.

### (7. Supplemental Remarks)

Embodiments of the present disclosure encompass an information processing apparatus (client apparatus or server apparatus) and system as described in the foregoing, an information processing method executed by an information processing apparatus or system, a program for causing an information processing apparatus to function, and a non-transitory computer readable medium storing such a program, for example.

The preferred embodiments of the present disclosure have been described above with reference to the accompanying drawings, whilst the present disclosure is not limited to the above examples, of course. A person skilled in the art may find various alterations and modifications within the scope of the appended claims, and it should be understood that they will naturally come under the technical scope of the present disclosure.

In addition, the effects described in the present specification are merely illustrative and demonstrative, and not limitative. In other words, the technology according to the present disclosure can exhibit other effects that are evident to those skilled in the art along with or instead of the effects based on the present specification.

Additionally, the present technology may also be configured as below.
(1) An information processing apparatus including:
   a stable section detection unit configured to detect a stable section of a posture of a sensor device that a user wears, based on first sensor data provided from the sensor device;
   a specific action section detection unit configured to detect a specific action section in which a specific action of the user occurs, based on second sensor data; and
   a sleep section detection unit configured to detect a sleep section in which the user is in a sleep state, based on a relationship between the stable section and the specific action section.
(2) The information processing apparatus according to (1),
   wherein the stable section is a section in which the posture of the sensor device is unchanged.
(3) The information processing apparatus according to (2),
   wherein the specific action section is a section in which a posture of the user is changed, and
   wherein in a case where the stable section and the specific action section are repeated, the sleep section detection unit detects a sleep section that includes the stable section and the specific action section.
(4) The information processing apparatus according to (3),
   wherein both the first sensor data and the second sensor data include acceleration sensor data.
(5) The information processing apparatus according to (1),
   wherein the stable section is a section in which the posture of the sensor device is regularly changed.
(6) The information processing apparatus according to (5),
   wherein the specific action section is a section in which an image or a voice of the user shows a characteristic associated with a sleep, and
   wherein in a case where the stable section and the specific action section are concurrent, the sleep section detection unit detects a sleep section that includes the stable section.
(7) The information processing apparatus according to (6),
   wherein the first sensor data includes acceleration sensor data,
   wherein the second sensor data includes image data, and
   wherein the specific action section is a section in which it is recognized that the user closes eyes based on the image data.
(8) The information processing apparatus according to (6),
   wherein the first sensor data includes acceleration sensor data,
   wherein the second sensor data includes audio data, and
   wherein the specific action section is a section in which it is recognized that the user emits a specific sound based on the audio data.
(9) The information processing apparatus according to any one of (1) to (8),
   wherein the sleep section detection unit detects the sleep section further based on user behavior that is recognized based on sensor data different from the first and second sensor data.
(10) The information processing apparatus according to any one of (1) to (9), further including:
   an external device control unit configured to output a control signal of an external device in a case where a start of the sleep section is detected.
(11) The information processing apparatus according to (10),
   wherein the external device control unit outputs a control signal for stopping or pausing the external device.
(12) The information processing apparatus according to (10),
   wherein the external device control unit outputs the control signal for causing the external device to set an alarm based on a start time of the sleep section.
(13) The information processing apparatus according to any one of (1) to (12), further including:
   a notification information output unit configured to output information for notifying another user of a detection result of the sleep section.
(14) The information processing apparatus according to (13),
   wherein the notification information output unit outputs information indicating a start, a continuation, or an end of the sleep section.
(15) The information processing apparatus according to (13),
   wherein the notification information output unit outputs information indicating a length of the sleep section in a predetermined period.
(16) An information processing method including:
   detecting a stable section of a posture of a sensor device that a user wears, based on first sensor data provided from the sensor device;
   detecting a specific action section in which a specific action of the user occurs, based on second sensor data; and
   detecting, by a processor, a sleep section in which the user is in a sleep state, based on a relationship between the stable section and the specific action section.
(17) A program for causing a computer to execute:
   a function of detecting a stable section of a posture of a sensor device that a user wears, based on first sensor data provided from the sensor device;
   a function of detecting a specific action section in which a specific action of the user occurs, based on second sensor data; and
   a function of detecting a sleep section in which the user is in a sleep state, based on a relationship between the stable section and the specific action section.

### Reference Signs List

- 10, 20: system
- 100: sensor device
- 110: sensor
- 120: pre-processing unit
- 130: memory
- 200: smartphone
- 210: position detection unit
- 220: sensor
- 230: behavior recognition unit
- 240: processing unit
- 250: integrated analysis unit
- 260: storage
- 270: application
- 300: information processing apparatus
- 301: acquisition unit
- 303: stable section detection unit
- 305: specific action section detection unit
- 307: sleep section detection unit
- 309: external device control unit
- 311: notification information output unit

## Claims

1. An information processing apparatus comprising:
a stable section detection unit configured to detect a stable section of a posture of a sensor device that a user wears, based on first sensor data provided from the sensor device;
a specific action section detection unit configured to detect a specific action section in which a specific action of the user occurs, based on second sensor data; and
a sleep section detection unit configured to detect a sleep section in which the user is in a sleep state, based on a relationship between the stable section and the specific action section.

2. The information processing apparatus according to claim 1,
wherein the stable section is a section in which the posture of the sensor device is unchanged.

3. The information processing apparatus according to claim 2,
wherein the specific action section is a section in which a posture of the user is changed, and
wherein in a case where the stable section and the specific action section are repeated, the sleep section detection unit detects a sleep section that includes the stable section and the specific action section.

4. The information processing apparatus according to claim 3,
wherein both the first sensor data and the second sensor data include acceleration sensor data.

5. The information processing apparatus according to claim 1,
wherein the stable section is a section in which the posture of the sensor device is regularly changed.

6. The information processing apparatus according to claim 5,
wherein the specific action section is a section in which an image or a voice of the user shows a characteristic associated with a sleep, and
wherein in a case where the stable section and the specific action section are concurrent, the sleep section detection unit detects a sleep section that includes the stable section.

7. The information processing apparatus according to claim 6,
wherein the first sensor data includes acceleration sensor data,
wherein the second sensor data includes image data, and
wherein the specific action section is a section in which it is recognized that the user closes eyes based on the image data.

8. The information processing apparatus according to claim 6,
wherein the first sensor data includes acceleration sensor data,
wherein the second sensor data includes audio data, and
wherein the specific action section is a section in which it is recognized that the user emits a specific sound based on the audio data.

9. The information processing apparatus according to claim 1,
wherein the sleep section detection unit detects the sleep section further based on user behavior that is recognized based on sensor data different from the first and second sensor data.

10. The information processing apparatus according to claim 1, further comprising:
an external device control unit configured to output a control signal of an external device in a case where a start of the sleep section is detected.

11. The information processing apparatus according to claim 10,
wherein the external device control unit outputs a control signal for stopping or pausing the external device.

12. The information processing apparatus according to claim 10,
wherein the external device control unit outputs the control signal for causing the external device to set an alarm based on a start time of the sleep section.

13. The information processing apparatus according to claim 1, further comprising:
a notification information output unit configured to output information for notifying another user of a detection result of the sleep section.

14. The information processing apparatus according to claim 13,
wherein the notification information output unit outputs information indicating a start, a continuation, or an end of the sleep section.

15. The information processing apparatus according to claim 13,
wherein the notification information output unit outputs information indicating a length of the sleep section in a predetermined period.

16. An information processing method comprising:
detecting a stable section of a posture of a sensor device that a user wears, based on first sensor data provided from the sensor device;
detecting a specific action section in which a specific action of the user occurs, based on second sensor data; and
detecting, by a processor, a sleep section in which the user is in a sleep state, based on a relationship between the stable section and the specific action section.

17. A program for causing a computer to execute:
a function of detecting a stable section of a posture of a sensor device that a user wears, based on first sensor data provided from the sensor device;
a function of detecting a specific action section in which a specific action of the user occurs, based on second sensor data; and
a function of detecting a sleep section in which the user is in a sleep state, based on a relationship between the stable section and the specific action section.
